# EUROPEAN PATENT APPLICATION

(11) **EP 1 649 842 A1**
(43) Date of publication of application: **26.04.2006**
(21) Application number: 05256601.5
(22) Date of filing: 25.10.2005
(51) Int. Cl.: A61F 7/10

(54) **Method and apparatus for insulating body organs during transplantation**

(30) Priority: 25.10.2004 US 972758
(71) Applicant: Thompson Surgical Instruments, Inc., Traverse City MI 49684 (US)
(72) Inventor: Kansoul, Hassan A. Huddinge University, Stockholm 14186 (SE); Farley, Daniel K., Traverse City, Michigan 49684 (US); Mulac, Antony J., Traverse City, Michigan 49684 (US)
(74) Representative: Hitchcock, Esmond Antony

(57) **Abstract**

A method and apparatus for thermally affecting graft organs during harvesting and transplantation operations. An insulation jacket (10) is configured to conform to the shape of the graft organ. The insulation jacket includes a body portion (11) constructed from a flexible surgical grade plastic, insulation foam, or thermo mass. The body portion may include a plurality of non-communicating pillows (22) capable of retaining a malleable condition when chilled to desired implantation temperatures. The selection of material for the body portion and the sterile nature of the cooling material allow the insulation jacket to be placed inside a patient's body during surgery. When secured about a graft organ, the insulation jacket provides openings for access to graft organ vessels, thereby allowing the implantation surgery to proceed while at least a portion of the graft organ remains enclosed in the insulation jacket. The insulation jacket may also include at least one layer of insulation.

## Description

The present invention pertains to a method and apparatus for thermally affecting graft organs during harvesting and transplantation procedures. More particularly, the present invention pertains to an insulation jacket that may cool graft organs during harvesting procedures and may insulate the graft organ from heat sources during transplantation.

Transplantation surgery is one of the leading and fastest growing surgical technologies of our time. The rapid development of this field is due to current organ donation policies, changes in public awareness and viewpoints pertaining to the necessity for donors, and recent technical innovations that are making transplantations easier and safer to perform. While technological improvements have reduced some of the complications associated with transplantation surgery, other severe complications continue to exist.

One of the most frequent complications in transplantation surgeries is ischemia. Ischemia is the reduction or stoppage of flow of oxygen and nutrients to living cells. Ischemia may have drastic consequences, including apoptosis and senescence, which entails the death of the oxygen and nutrient deprived cells. Unfortunately, because the supply of blood and nutrients ceases when a graft organ is removed from a donor's body, ischemia is always present in transplantation procedures.

Ischemia has been the central focus of numerous research projects and studies. As a result of these studies, many theories have been developed to better explain the detection and consequences of ischemia and to suggest how to slow down its damaging effects. Previous attempts to limit the consequences of ischemia, and thereby improve graft organ preservation, include training centers for surgeons devoted to reducing operation time and improving implantation techniques, the development of drugs designed to protect cells from entering into apoptosis, and the cooling of donated graft organs prior to implantation.

From these studies and projects, two types of ischemia have been defined, namely warm ischemia and cold ischemia. Warm ischemia typically begins when the blood supply to the organ is stopped and the graft organ is removed from the donor's body. The onset of apoptosis during warm ischemia typically occurs extremely fast and leads to irreversible damages. Injury due to warm ischemia has had a severe influence on the viability and post-transplantation outcome of organ grafts. However, after many years of research, it was discovered that the apoptosis process could be slowed down by reducing the temperature of the graft organ, which is known as cold ischemia.

Cold ischemia reduces the temperature of the removed graft organ so that enzymatic activity is delayed or stops altogether. The slowing of the apoptosis process through the use of cold ischemia significantly reduces the presence of irreversible complications. For example, while exposure of the graft organ to warm ischemia is measured in minutes, cold ischemia is measured in hours. Thus, the discovery of the benefits of cold ischemia over warm ischemia was a big step towards improving the success of transplantation procedures.

Yet, injuries due to cold and warm ischemia remain an important source of morbidity and mortality in some transplantation procedures. For instance, while cold ischemia may slow the apoptosis process, the graft organ may be irreparably damaged if cooled below 4° Celsius. Further, similar to warm ischemia, there is a time limit on how long a graft organ may withstand cold ischemia.

However, because of the benefits offered by cold ischemia, graft organs are prepared for transplantation in a cold environment. A graft organ is typically reduced to a temperature of approximately 4° Celsius. However, during the implantation operation, the temperature of the graft organ gradually increases and results in the onset of undesirable warm ischemia. This increase in temperature may be facilitated by the exposure of the graft organ to a number of different heat sources, including warmth from the recipient's body, the surgeon's hands, operating lights, general room illumination, room temperature, and operating instruments.

Many existing cooling packs are designed to cool a variety of items, including food, beverages, and biological materials, such as organs. Such cooling packs may be similar to ice packs in which a sealed pouch is filled with a thermal cooling agent that is converted to a frozen solid state when cooled, whereupon the cooling agent may no longer be malleable. Other prior art devices include a thermal cooling agent that does not transform into a solid state when cooled. However, these thermal cooling agents are often contain within a single bladder-like enclosure or a series of individual chambers that are in communication with adjacent chambers. The problem with such configurations is that if one section of the bladder or a single chamber is accidentally or purposefully breached, a significant portion, if not all, of the cooling agent may flow out of the cooling pack, and thus hinder or ruin the ability of the cooling pack to function.

Further, prior art cooling packs may have a general, non-organ specific shape or configuration. Unfortunately, in organ transplantation and harvesting operations, the general shape of a cooling pack may result in unnecessary interference with the field of operation.
Additionally, by not providing a cooling pack that is configured for use with a specific type of graft organ, the prior art devices may not, when at least partially secured about a graft organ, provide openings through which graft organ vessels or veins may pass away from the graft organ and cooling pack. The lack of openings may prevent the surgeon from having access to necessary vessels and veins during a harvesting or transplantation procedure.

The present invention is an insulation jacket for thermally affecting a graft organ during harvesting and transplantation operations. In principle embodiment, the jacket comprises;
a) a body portion having an inner wall and an outer wall, the inner wall operably connected to the outer wall to form a plurality of pillows, the plurality of pillows having an inner section, the plurality of pillows not being in communication with adjacent pillows, the body portion generally configured in the shape of the graft organ;
b) a cooling material located within the inner section, the cooling material being non-toxic and sterile, the cooling material contained within the plurality of pillows being capable of having a malleable nature when cooled to approximately 4 degrees Celsius; and
c) a plurality of connectors, the plurality of connectors operably connected to the insulation jacket, the plurality of connectors being configured to secure the insulation jacket about at least a portion of the graft organ.

In an insulation jacket of the invention, at least one layer of insulation material may be operably attached to the body portion. The jacket may include at least one of an outer layer of insulation operably connected to the outer wall of the body portion, and an inner layer of insulation operably connected to the inner wall of the body portion. Typically, at least one of the layers of insulation is comprised of a closed cell insulating foam.

Preferred embodiments of the invention include a plurality of interconnecting bands positioned between the plurality of pillows, configured to assist in the ability of the insulation jacket to be manipulated about at least a portion of the graft organ. In another preferred feature, the body portion is configured to form at least one opening when enclosed about at least a portion of the graft organ, said at least one opening positioned to allow at least one vessel of the graft organ to pass out of the insulation jacket. In all embodiments the body portion is preferably comprised of a plurality of panels.

In an alternative embodiment of the invention, the insulation jacket comprises;
a) a body portion having an inner wall and an outer wall, the body portion generally configured in the shape of the graft organ, the body portion configured to generally conform to the shape of said graft organ when said body portion is secured about at least a portion of said graft organ, the body portion also configured to provide an opening through which at least one vessel of said graft organ may extend away from said body portion when said body portion is enclosed about at least a portion of the graft organ; and
b) a plurality of connectors, the plurality of connectors operably connected to the insulation jacket, the plurality of connectors being configured to secure the insulation jacket about at least a portion of the graft organ.

The body portion may be comprised of an insulation foam such as a closed cell foam; a thermo mass; or a surgical grade plastic. The jacket will normally include a liner, the liner and body portion being configured to form a plurality of pillows configured to retain a cooling material, wherein the body portion is configured to prevent said cooling material from leaking through said body portion.

In this alternative embodiment, at least one of the inner and outer walls of the body portion will normally be operably connected to at least one layer of insulation. In another preferred feature, the inner wall is operably connected to the outer wall to form a plurality of non-communicating pillows, the plurality of non-communicating pillows having an inner section, and being configured to contain a cooling material, which cooling material is non-toxic and sterile, and capable of having a malleable nature when cooled to approximately 4 degrees Celsius.

Pursuant to the above, the present invention seeks to enable prevention or delay of the warrning of a graft organ during an implantation process. The invention also seeks to provide techniques for insulating a graft organ placed inside a recipient's body during a transplantation operation and thereby prevent the onset of warm ischemia. The organ insulation jacket can provide a surgeon with access to required vessels of the graft organ during transplantation surgery while the insulation jacket continues to enclose at least a portion of the graft organ. The jacket may include a non-toxic and sterile cooling material to reduce the temperature of the graft organ , the material being able to retain a malleable nature when cooled to desired implantation temperatures.

The present invention pertains to a method and apparatus for insulating graft organs during harvesting and transplantation procedures. More particularly, the present invention pertains to an insulation jacket that may enclose at least a portion of a graft organ and which may be sterilized so as to be capable of being placed within the body of a patient during a harvesting and transplantation operation. The insulation jacket includes a body portion that may be constructed from a flexible surgical grade plastic, insulation foam, or a thermo mass, for example a dense gel. The body portion may also be operably connected to connectors that allow the body portion to be secured in a closed position when manipulated about at least a portion of a graft organ. In one embodiment of the present invention, the body portion may be configured to form a plurality of pillows, the pillows being configured to contain a non-toxic sterile cooling material. In such an embodiment, the cooling material may assist in reducing the temperature of the graft organ to desired transplantation temperatures. Further, some embodiments of the present invention may also include at least one layer of insulation that may assist in insulating the graft organ and/or cooling materials from outside heat sources. The selection of materials for the body portion and, when used, the sterile nature of the cooling material allows the insulation jacket to be placed inside a patient's body during harvesting and transplantation surgery.

The body portion includes inner and outer walls and may be specifically shaped for a particular type of organ and its attached veins and vessels, such as, but not limited to, a heart, liver, lung, pancreas, and kidney. The body portion may be comprised of at least one panel. The panel may be operably connected to an adjacent panel through the use of an adhesive. The body portion may also be configured so that, when enclosed about at least a portion of the graft organ, the insulation jacket includes openings which may be positioned around vessels and arteries of the graft organ so that the vessels may pass through or extend away from the insulation jacket. These openings in the insulation jacket provide the surgeon with access to organ vessels, which allows the surgeon to conduct the transplantation surgery while at least a portion of the graft organ remains enclosed in the insulation jacket

The non-toxic sterile cooling material may include, but is not limited to, a sterile liquid or gel. The selected cooling material may be capable of retaining a malleable condition when cooled to a temperature of approximately 4° Celsius. Each of the plurality of pillows may not be in communication with adjacent pillows, and thus the puncture or rupture of one pillow may not result in the loss of cooling material from adjacent pillows. Therefore, in the event that a pillow is punctured, tom, or ruptures, adjacent pillows may still retain the chilled or unchilled cooling material.

Further, the selection of materials for the body portion may also allow the body portion to have an elastic nature. For example, the flexible nature of some surgical grade plastics may allow at least some areas of the body portion between the non-communicating pillows to function as elastic bands. Besides connecting adjacent pillows, the flexible nature of any such elastic bands could assist in the ability of the insulation jacket to be manipulated about the specific graft organ that the insulation jacket was designed to at least partially enclose.

In some embodiments of the present invention, the body portion may be operably connected to at least one layer of insulation. The insulation may be operably affixed along at least a portion of the inner wall and/or outer wall of the body portion of the insulation jacket. Also, the layer of insulation may be constructed from a number of different materials, including, but not limited to, a closed cell insulating foam, including a polyethylene closed cell foam. Further, the layer of insulation may be thin and malleable in nature so as to not interfere with the flexibility of the body portion. The layer of insulation may also serve as a wall of the body portion.

When used, the insulation jacket may be placed around the organ before transplantation into the recipient, including prior to harvesting the graft organ from the donor's body. For example, when possible, to further attempt to minimize the onset of warm ischemia, it may be preferable to not cut the vessels supplying blood and oxygen to the harvested organ until the insulation jacket has been placed around the graft organ. Once secured around the graft organ, the graft organ may be removed from the donor's body. The graft organ may then be prepared for implantation into the body of the recipient.

While being prepared for transplantation, the organ is preferably maintained at approximately 4° Celsius. During the transplantation operation, the graft organ may remain enclosed at least in part by the insulation jacket so as to prevent damage to the cells that is associated with warm ischemia. For embodiments of the present invention that include a plurality of pillows, because the pillows may not be in communication with adjacent pillows, the loss of cooling material in pillows that are damaged (i.e. pillows that may be ruptured, punctured, torn, severed, or pierced) may not cause undamaged adjacent pillows to lose any of their cooling material. Therefore, the surgeon may accidentally or purposely remove cooling material from some pillows without completely destroying the ability of the insulation jacket to continue to cool the graft organ.

Embodiments of the invention will now be described by way of example and with reference to the accompanying drawings wherein:

Figure 1 illustrates an inside elevation view of an insulation jacket in accordance with one embodiment of the present invention.

Figure 2 illustrates an outside elevation view of an insu lation jacket configured to enclose at least a portion of a graft organ in accordance with one embodiment of the present invention.

Figure 3 illustrates a cross sectional view of the pillows of an insulation jacket in accordance with one embodiment of the present invention.

Figure 4 illustrates an outside elevation view of an insulation jacket having a plurality of panels and connectors in which the insulation jacket is secured about at least a portion of a graft organ in accordance with one embodiment of the present invention.

Figure 5 illustrates a partial, cross-sectional end view of an insulation jacket in accordance with one embodiment of the present invention.

Figure 6 illustrates an outside elevation view of an insulation jacket having an insulated outer wall in accordance with one embodiment of the present invention.

Figure 7 illustrates a cross sectional view of an insulation jacket having an insulated outer wall in accordance with one embodiment of the present invention.

Figure 8 illustrates of an outside elevation view of an insulation jacket having a plurality of panels and connectors in which the insulation jacket is secured about at least a portion of a graft organ in accordance with one embodiment of the present invention.

Figure 9 illustrates a partial, cross-sectional end view of an insulation jacket having insulation on the outer wall in accordance with one embodiment of the present invention.

Figure 10 illustrates an inside elevation view of an insulation jacket having an insulated outer wall and an inner wall that is not insulated in accordance with one embodiment of the present invention embodiment of the present invention.

Figure 11 illustrates an outside elevation view of an insulation jacket having an insulated outer wall and an insulated inner wall in accordance with one embodiment of the present invention.

Figure 12 illustrates a cross sectional view of the insulation jacket that is insulated on both the inner wall and outer wall in accordance with one embodiment of the present invention.

Figure 13 illustrates of an outside elevation view of an insulation jacket having a plurality of panels and connectors in which the insulation jacket is secured about at least a portion of a graft organ in accordance with one embodiment of the present invention.

Figure 14 illustrates a partial, cross-sectional end view of an insulation jacket having insulation on both the inner wall and outer wall in accordance with one embodiment of the present invention.

Figure 15 illustrates an inside elevation view of insulation jacket having an insulated inner and outer wall in accordance with one embodiment of the present invention.

Figure 16 illustrates an inside elevation view of an insulation jacket in accordance with one embodiment of the present invention in which the body portion is comprised of an insulation foam.

Figure 17 illustrates an outside elevation view of an insulation jacket configured to enclose at least a portion of a graft organ in accordance with one embodiment of the present invention in which the body portion is comprised of an insulation foam.

Figure 18 illustrates a cross sectional view of the closed cells of an insulation jacket in accordance with one embodiment of the present invention in which the body portion is comprised of an insulation foam.

Figure 19 illustrates of an outside elevation view of an insulation jacket having a plurality of panels and connectors in which the insulation jacket is secured about at least a portion of a graft organ in accordance with one embodiment of the present invention in which the body portion is comprised of an insulation foam.

Figure 20 illustrates an inside elevation view of an insulation jacket in accordance with one embodiment of the present invention in which the body portion is comprised of a thermo mass.

Figure 21 illustrates an outside elevation view of an insulation jacket configured to enclose at least a portion of a graft organ in accordance with one embodiment of the present invention in which the body portion is comprised of a thermo mass.

Figure 22 illustrates of an outside elevation view of an insulation jacket having a plurality of panels and connectors in which the insulation jacket is secured about at least a portion of a graft organ in accordance with one embodiment of the present invention in which the body portion is comprised of a thermo mass.

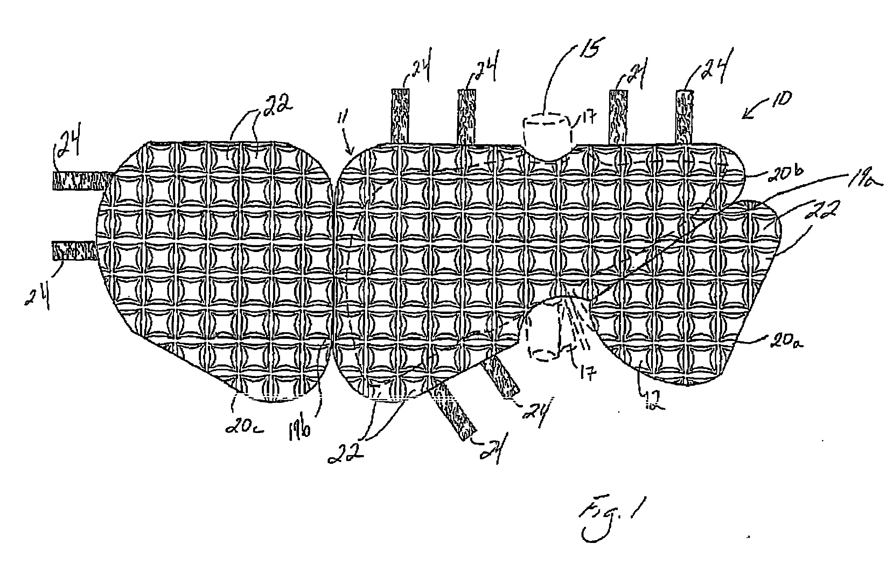

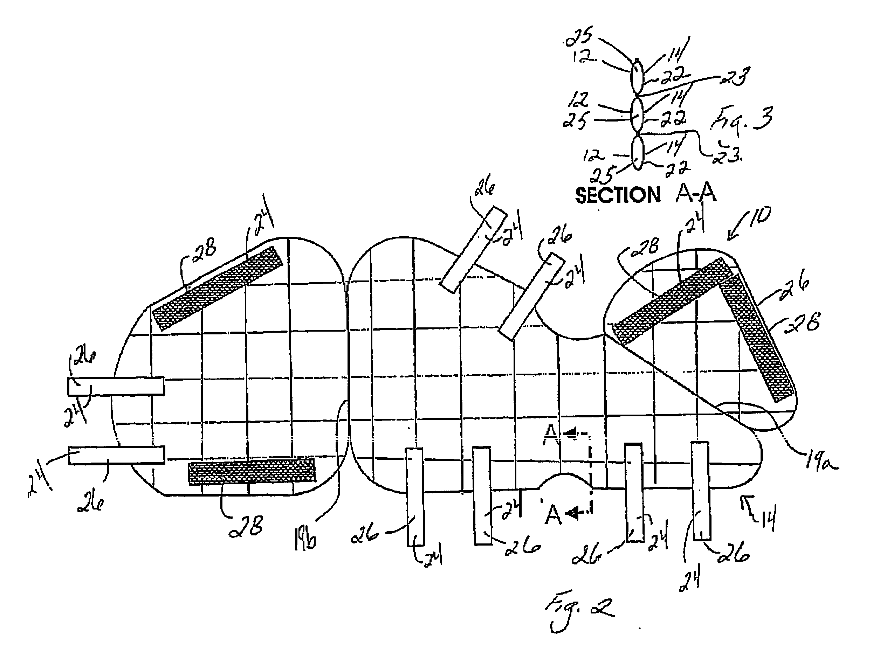

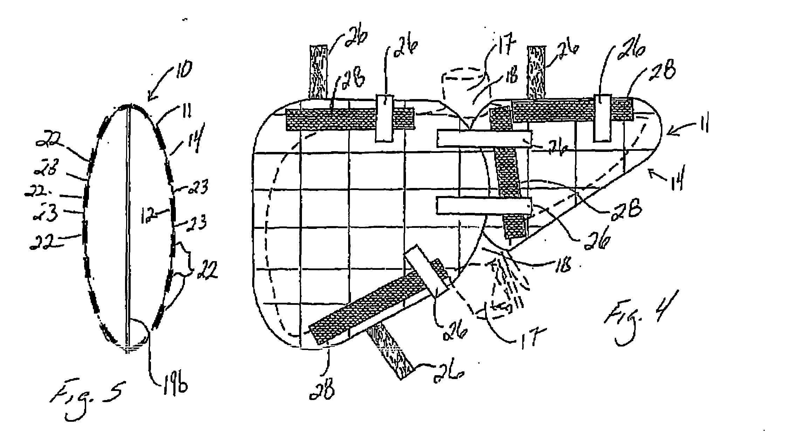

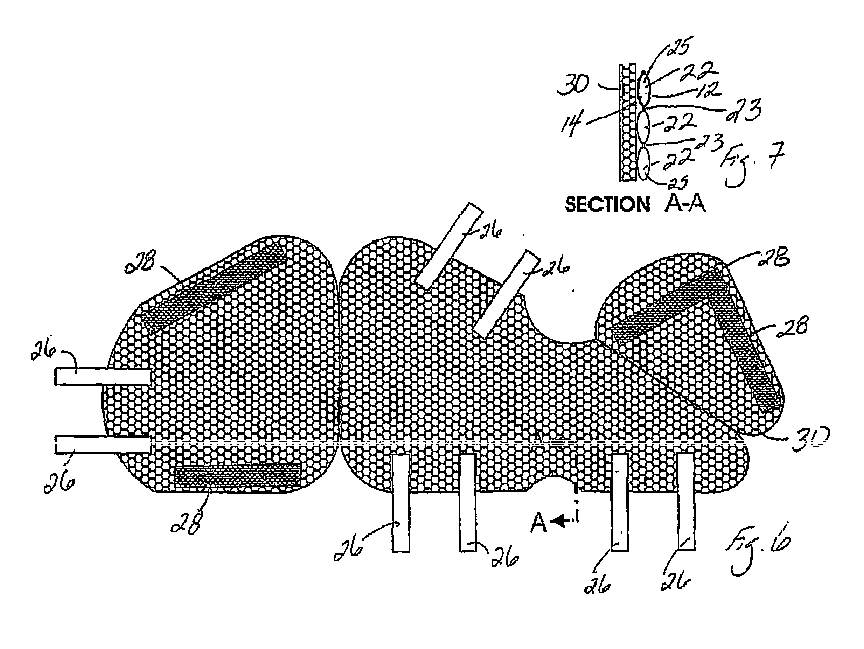

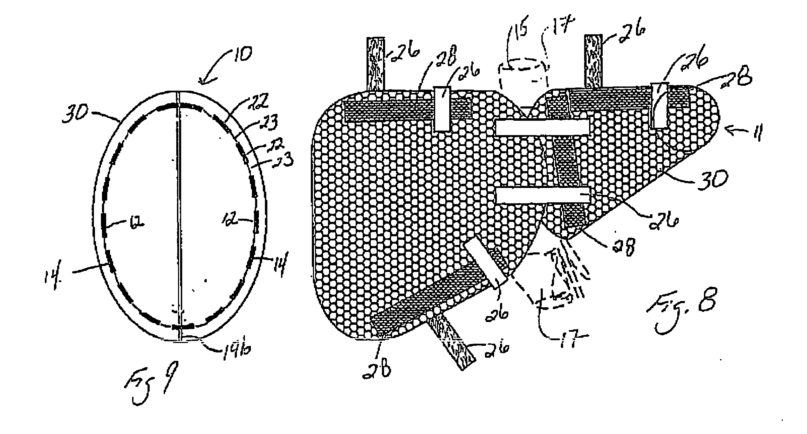

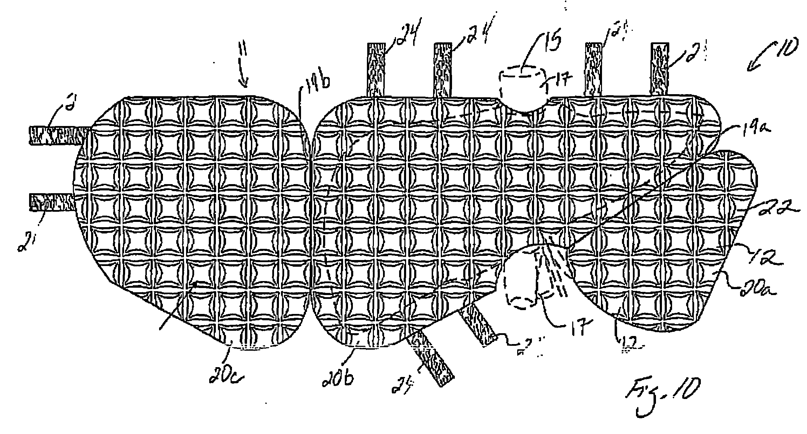

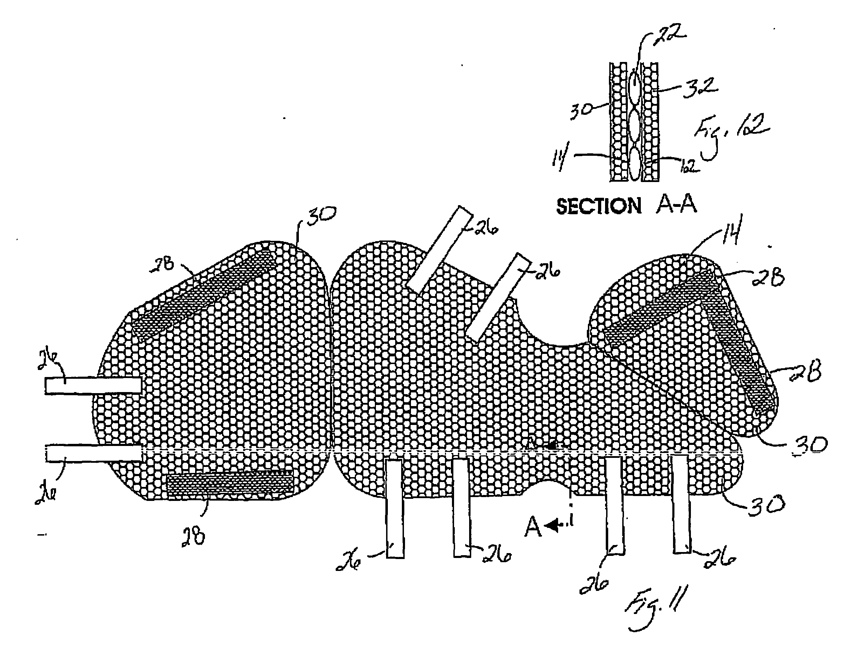

Figure 1 illustrates an inside elevation view of an insulation jacket 10 that is configured to enclose at least a portion of a graft organ 15 in accordance with one embodiment of the present invention. The insulation jacket 10 may include a body portion 11 and a plurality of connectors. As shown in Figures 1 and 2, the body portion 11 has an inner wall 12 and an outer wall 14 and may be constructed from relatively thin and flexible surgical grade plastics. Further, the inner wall 12 and outer wall 14 may be separate pieces of flexible surgical grade plastics that are operably connected, such as through the use of adhesive materials, to form the body portion 11. The size and shape of the body portion 11 may be determined by the specific type of graft organ 15. More specifically, the insulation jacket 11 may be contoured and sized to enclose at least a portion of a specific organ, for example a heart, liver, lung, or kidney, while still providing access to the vessels 17 of the graft organ *15*.

For example, as is known, different types of graft organs have different shapes and sizes. More specifically, although the actual size of a graft organ may vary from person to person, generally, a normal adult liver may be 28 cm long, 8 cm in height, and 18 cm in antero-posterior thickness. Further, an average kidney in a living adult may weigh from 2000 to 2500 grams, while the liver in a cadaver may weigh 1400 to 1500 grams. Although the actual dimensions of the kidney of an adult may vary depending on a variety of factors, including sex, age, and pathology, a normal adult kidney may be 10 to 12 cm long vertically, 5 to 6 cm wide, have a 3 cm antero-postenor thickness, and weigh 115 to 150 grams. Additionally, while a variety of factors may also affect the size of a lung, a lung removed from an adult of intermediary respiratory status may have a vertical length of 25 cm, a width at the base of 15 cm, a transversal base measurement of 10 cm, and a weight of 700 grams. Therefore, by designing the shape of the body portion 11 to cover at least a portion of specific type of graft organ *15*, the organ specific body portion 11 may occupy a minimal amount of space in the operation field and thus may minimize the potential risk that the insulation jacket 10 may interfere with the vision or maneuverability of the surgeon during an implantation operation.

While the body portion 11 may be a single panel 20 that is designed to encompass at least a portion of a graft organ *15*, in the illustrated embodiment, the body portion 11 may be comprised of a plurality of panels 20a, 20b, 20c that are operably connected to an adjacent panel. In such an embodiment, the panels 20a, 20b, 20c maybe operably connected to an adjacent panel through the use of an adhesive. The location of the attachment of panels 20a, 20b, 20c may result in the formation of creases 19a, 19b between adjacent panels 20a, 20b, 20c. Because the insulation jacket 10 may be designed for use with a specific type of graft organ *15,* the creases 19a, 19b may be positioned in locations in which the body portion 11 is folded about at least a portion of the graft organ, thereby assisting in preventing the formation of undesirable protruding points and corners that may harm adjacent tissue or take up additional space in the operating field.

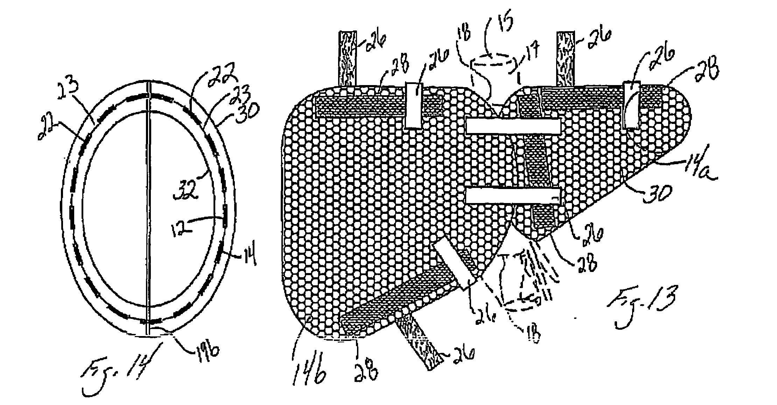

Figure 3 illustrates a cross sectional view the pillows of an insulation jacket 10 in accordance with one embodiment of the present invention. As illustrated in Figure 3, the inner wall 12 and outer wall 14 of the body portion 11 may be configured to allow for the formation of a plurality of pillows 22. The pillows 22 may include an inner section 25 that is configured to contain a cooling material. The cooling material may be a sterile liquid or gel that will not freeze solid when cooled to temperatures of at least approximately 40 Celsius and which will remain malleable at low temperatures. Suitable cooling material include, but are not limited to, Aquasonic™ Clear ultrasound gel from Parker Laboratories Inc. of Fairfield, New Jersey, lubricants such as K-Y™ lubricants offered by a division of McNeil-P.P.C., Inc. of Skiliman, New Jersey, a Johnson and Johnson company, and saline solutions.

In the illustrated embodiment, the pillows 22 may not be in communication with other adjacent pillows 22, but instead may be separated so that the tearing or puncturing of one pillow 22 will not result in the loss of cooling material from an adjacent undamaged pillow 22. In the illustrated embodiment, the connection of the inner wall 12 and outer wall 14 across the interconnecting region between adjacent non-communicating pillows 22 allows for the formation of bands 23. Because of the flexible nature of the material used for the body portion 11, the bands 23 may be elastic in nature and thus may assist in providing flexibility and/or plasticity to the body portion 11.

Figure 2 illustrates an outside elevation view of an insulation jacket 10 configured in accordance with one embodiment of the present invention. Connectors may be used to secure the body portion 11 around at least a portion of the graft organ *15*. The connectors may include, but are not limited to, mating strips of hook and loop material, staples, tape, and adhesives.

Figure 4 illustrates an outside elevation view of an insulation jacket 10 having a plurality of panels 20a, 20b, 20c and connectors in which the insulation jacket is secured about at least a portion of a graft organ in accordance with one embodiment of the present invention. As shown, the connectors may include mating strips of hook and loop material in which a first strip 26 is positioned to attach to a mating second strip 28 when the body portion 11 is partially secured around a graft organ *15*. Further, in the embodiment of the present invention illustrated in Figures 1, 2, and 4, the connectors may be operably attached to the outer wall 14 of the body portion 11, including through the use of an adhesive material.

As also illustrated in Figure 4, the contours and shape of the body portion 11 allow the insulation jacket 10 to enclose at least a portion of the graft organ 15 while still providing openings 18 for the vessels 17 and/or arteries of the graft organ 15 to pass out of the insulation jacket 10. By providing these openings 18, a surgeon may have access to the main vessels 17 and/or veins while the insulation jacket 10 is placed around at least a portion of the graft organ 15 during the removal and/or subsequent transplantation of the graft organ *15*.

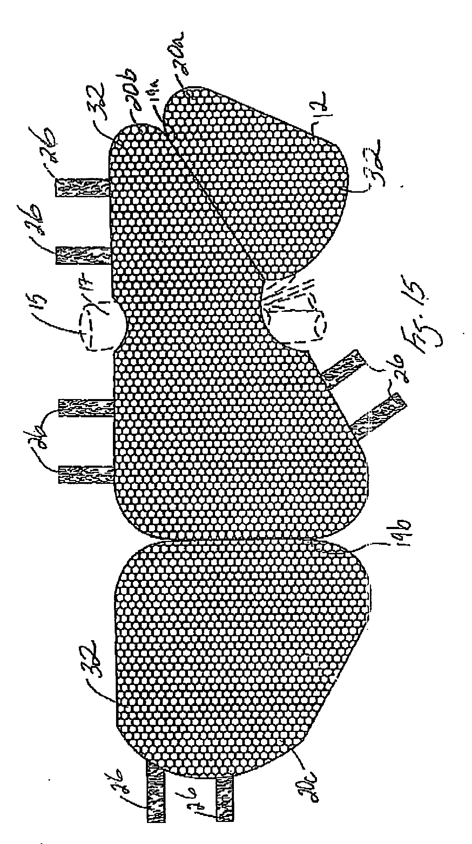

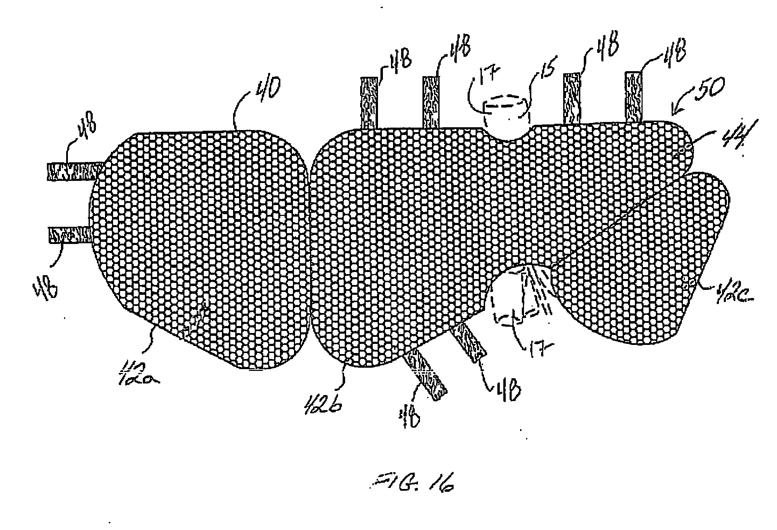

Figure 5 illustrates a partial, cross-sectional end view of an insulation jacket 10 of Figure 4. When wrapped about at least a portion of a graft organ (not shown), the surface of the pillows 22 along the inner wall 12 may come into direct contact with at least a portion of the enclosed graft organ. In such an embodiment, because of the potential direct physical contact between the surface of the pillows 22 along the inner wall 12 and the graft organ, the temperature of the cooling material contained within the pillows 22 should not be reduced to temperatures that may cause damage to the graft organ. More particularly, the temperature of the pillows 22 and cooling material contained therein preferably should not be cooled to be below 4° Celsius so as to ensure that the tissue and cells of the graft organ that come into contact with the pillows 22 are not unnecessarily damaged. [0057] Figures 6, 7, 8, 9, and 10 illustrate another embodiment of the present invention in which the insulation jacket 10 illustrated in Figures 1-5 also includes an outer layer of insulation 30 along at least a portion of the outer wall 14. The outer layer of insulation 30 may be operably attached to the body portion 11, including through the use of an adhesive. For example, the outer layer of insulation 30 may be adhered to the outer wall
14. Further, as shown, the outer layer of insulation 30 may also be operably connected to the first and second strips 26, 28 of the connectors, such as through the use of an adhesive material. Additionally, the outer layer of insulation 30 may be comprised of an insulation foam, including, but not limited to, a closed cell insulating foam, for example a polyethylene foam. Alternatively, the outer layer of insulation 30 may be comprised of multiple layers of sterile drapes that are encapsulated in a surgical grade material. By placing the outer layer of insulation 30 along the outer wall 14, the outer layer of insulation 30 may insulate the insulation jacket 10 from outside heat sources, including, but not limited to, heat from the body of the patient, surgical lights, and surgical equipment, and thereby assist in retaining the cool temperature of the cooling material and/or graft organ 15 for longer periods of time. Similarly, the layer of insulation may be positioned along a portion of the inner wall 12 of the insulation jacket 10 rather than being along the outer wall 14, as would be understood by one of ordinary skill in the art.

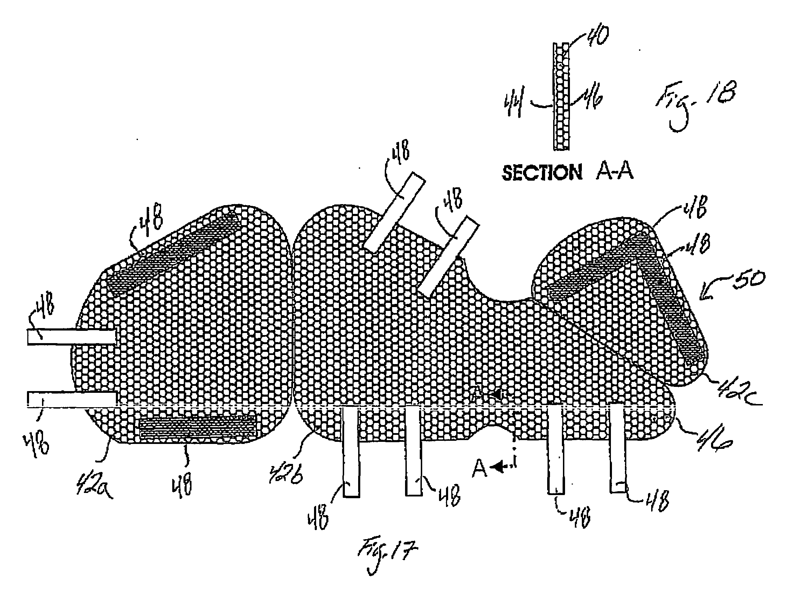

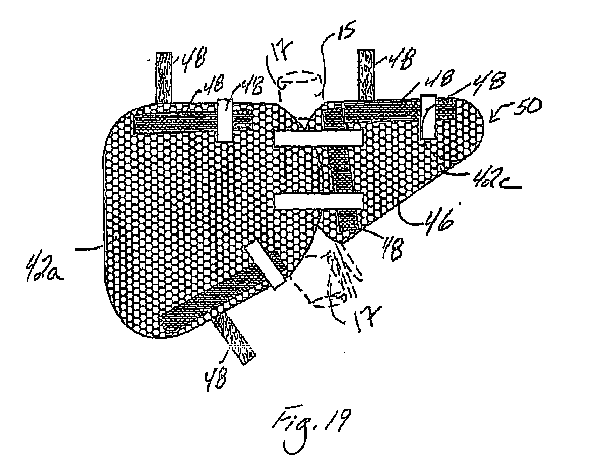

Figures 11, 12, 13, 14, and 15 illustrate yet another embodiment of the insulation jacket 10 illustrated in Figures 1-5 in which both the inner and outer walls 12, 14 are insulated. As previously discussed, at least a portion of the outer wall 14 may be operably connected to an outer layer of insulation 30 and may also be operably connected to the connectors. Similarly, at least a portion of the inner wall 12 may be operably connected to an inner layer of insulation 32, including through the use of an adhesive. As with the outer layer of insulation 30, the inner layer of insulation 32 may be comprised of an insulation foam, for example a closed cell foam, or alternatively, may be comprised of multiple layers of sterile drapes that are encapsulated in a surgical grade material.

By insulating both the inner and outer walls 12, 14, the cooling medium may be able to maintain desirable temperatures during organ harvesting and/or transplantation procedures for longer periods of time so as to prevent or delay the harmful affects of warm ischemia. For example, the inner layer of insulation 32 may prevent the warming of the cooling material contained within the plurality of pillows 22 from exposure to the body heat of a patient. Additionally, the presence of the inner layer of insulation 32 may allow for the temperature of the cooling material to be lower than what may typically be achieved in embodiments that do not include an inner layer of insulation 32, such as temperatures below approximately 40 Celsius. In such circumstances, because the inner layer of insulation 32 does not function as a cooling source, but instead is an insulator, the inner layer of insulation 32 is able to insulate the cooling material from outside heat sources and ambient temperatures while also acting as a buffer against the direct exposure of the graft organ 15 to the pillows 22 and the cooled cooling material contained therein. By acting as a buffer, the inner layer of insulation 32 may prevent damage to the graft organ 15 and its tissue that may otherwise occur from direct contact with the chilled pillows 22. In such embodiments, the inner layer of insulation 32 may reach a thickness of approximately one-half an inch.

The method for using the insulation jacket 10 of the present invention includes chilling the cooling material contained within the insulation jacket 10. One factor considered in determining the appropriate chilling temperature for the cooling material is what temperatures the graft organ may be exposed to without causing cell and tissue damage. As previously discussed, the cooling material may be chilled so that when the insulation jacket 10 encloses at least a portion of the graft organ 15, the temperature of the portion of the insulation jacket 10 that comes into direct contact with the graft organ 15 should not be chilled to below approximately 4° Celsius. However, if the insulation jacket 10 includes an inner layer of insulation 32, the cooling material may be exposed to lower chilling or even freezing temperatures than what may be acceptable for embodiments of the present invention that do not include an inner layer of insulation 32.

As illustrated in Figures 4, 8, and 13, the insulation jacket 10 may include openings 18 that allow the insulation jacket 10 to be placed around at least a portion of the graft organ 15 before the graft organ 15 is severed from the body of the donor. Further, when harvesting a graft organ 15, because the pillows 22 may not be in communication with adjacent pillows 22, the surgeon may elect to puncture some pillows 22 in order to improve the positioning and/or enclosure of the insulation jacket 10 about at least a portion of the graft organ 15 or to improve the field of operation without destroying the cooling capabilities of insulation jacket 10.

After preparing the graft organ 15 for transplantation, at least a portion of the graft organ 15 is enclosed by the insulation jacket 10 prior to the insertion of the graft organ 15 into the body of the recipient. During this aspect of the procedure, the openings 18 provide the surgeon with access to the vessels 17 of the graft organ 15 needed for reattaching the graft organ 15 in the body of the recipient. This access permits the insulation jacket 10 to continue enclosing at least a portion of the graft organ 15 during the transplantation surgery, and thus may prevent or delay the onset of warm ischemia. As with the harvesting process, during the transplantation procedure, because of the sterile non-toxic nature of the cooling material, the surgeon also may elect to puncture some of the pillows 22 without destroying the cooling capabilities of the insulation jacket 10 or harming the graft organ 15 or patient. Upon completion of the transplantation procedure, the insulation jacket 10 may be removed from the body of the patient. Given the nature of use, the insulation jacket may be used for only one transplantation operation.

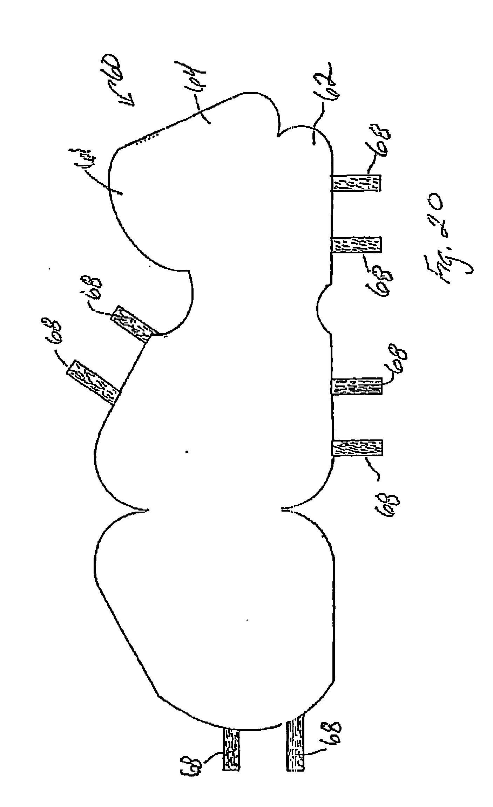

Figures 16 and 17 illustrate inside and outside elevation views of an insulation jacket 50 in accordance with alternative embodiment of the present invention in which insulation jacket 50 includes a body portion 40 that is comprised of an insulation foam, for example a polyethylene closed cell foam. The body portion 40 may have an inner wall 44 and an outer wall 46, as further shown in Figure 18. The body portion 40 may also be comprised of a single panel or a plurality of panels 42a, 42b, 42c, in which case the plurality of panels 42a, 42b, 42c may be operably connected to the adjacent panel, including being connected through the use of adhesives.

Figure 19 illustrates of an outside elevation view of an insulation jacket 50 having a plurality of panels 42a, 42b, 42c and connectors 48 in which the insulation jacket is secured about at least a portion of a graft organ in accordance with one embodiment of the present invention in which the body portion is comprised of an insulation foam. As shown, the insulation jacket 50 may be operably secured around at least a portion of the graft organ 15 through the use of connectors 48, including mating strips of hook and loop material, staples, tape, and adhesives. Additionally, the connectors 48 may be operably secured to the body portion 50, for example through the use of an adhesive material. Further, the body portion 40 may also be configured to generally conform to the shape of a specific type of a graft organ 15. By configuring the shape of the body portion 40 to generally conform to the shape of the graft organ 15, the insulation jacket 50 may have improved insulation characteristics while also minimizing the space in the operation field that is occupied by said insulation jacket 50 when the insulation jacket 50 is enclosed about at least a portion of the graft organ 15

In an alternative embodiment of the insulation jacket 50 illustrated in Figure 19, either the inner wall 44 or the outer wall 46 of the body portion 40 may be operably connected to a liner. For example, the liner, which may be constructed from a flexible surgical grade plastic, may be connected to at least a portion of the inner wall 44 or outer wall 46 through the use of an adhesive material. When connected to the inner wall 44 or the outer wall 46 of the body portion 40, the liner may be configured to form a plurality of non-communicating pillows between said liner and the inner wall 44 or outer wall 46. The pillows may contain a cooling material that may assist in cooling or retaining the chilled temperature of a graft organ. In such an embodiment, the selection of material for the body portion 40, for example the use of a closed cell foam, may prohibit cooling material contained within the pillows from leaking or seeping through the inner or outer walls 44, 46 of the body portion 40 respectively.

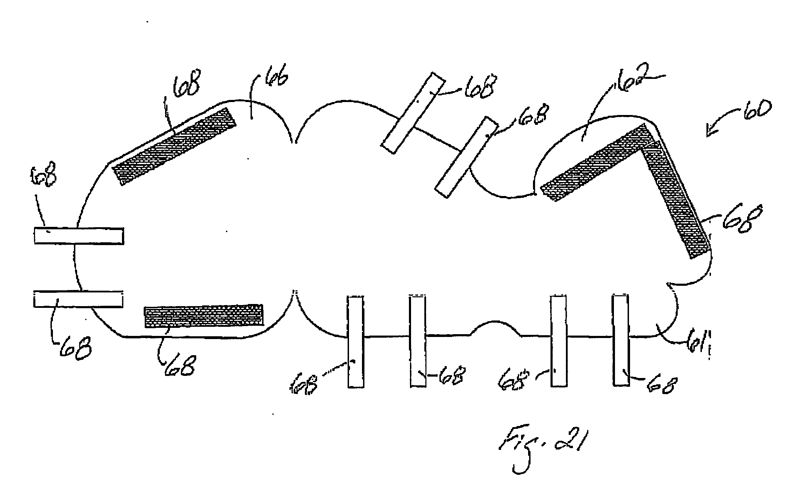

Figures 20 and 21 illustrate inside and outside elevation views of an insulation jacket 60 in accordance with another alternative embodiment of the present invention in which insulation jacket 60 includes a body portion 61 that is comprised of an thermo mass 62, for example a polyethylene closed cell foam. The thermo mass 62 may include an inner wall 64 and an outer wall 64 and may be comprised of, but is not limited to, a dense rubber thermo cc nductor gel, for example commercially available Akton™ Polymer from Action Products of Hagerstown, Maryland. In the illustrated embodiment, the thermo mass may be ¼ inch thick. In such an embodiment, the thermo mass 62 may have a sufficient density so that the thermo mass 62 may not have to be contained with in a separate bladder, while still being sufficiently plyable so as to allow the body portion 61 to be manipulated about at least a portion of the graft organ 15.

Figure 21 illustrates an outside elevation view of the insulation jacket 60 in which the insulation jacket 60 is secured about at least a portion of a graft organ 15. As with the previous embodiments, the thermo mass 62 may be shaped to conform to the shape of the graft organ 15 while still provide openings for the passage and/or access to the vessels 17 of the graft organ 15. Further, the body portion 61 may be operably connected to at least one connector 68, the connector 68 being configured to assist in securing the insulation jacket 60 about at least a portion of the graft organ 60. Additionally, the inner wall 64 and/or outer wall 66 of the body portion 61 may be operably connected to at least one layer of insulation, the at least one layer of insulation being configured to insulate the graft organ 15 from outside heat sources and assist in retaining the cool temperature of the chilled graft organ 15.

While the invention has been described with reference to certain embodiments, it will be understood by those skilled in the art that various changes may be made and equivalents may be substituted without departing from the scope of the invention. In addition, many modifications may be made to adapt a particular situation or material to the teachings of the invention without departing from its scope.

## Claims

1. An insulation jacket for thermally affecting a graft organ during harvesting and transplantation operations comprising:
a) a body portion having an inner wall and an outer wall, the inner wall operably connected to the outer wall to form a plurality of pillows, the plurality of pillows having an inner section, the plurality of pillows not being in communication with adjacent pillows, the body portion generally configured in the shape of the graft organ;
b) a cooling material located within the inner section, the cooling material being non-toxic and sterile, the cooling material contained within the plurality of pillows being capable of having a malleable nature when cooled to approximately 4 degrees Celsius; and
c) a plurality of connectors, the plurality of connectors operably connected to the insulation jacket, the plurality of connectors being configured to secure the insulation jacket about at least a portion of the graft organ.

2. An insulation jacket according to Claim 1 including at least one layer of insulation material operably attached to the body portion.

3. An insulation jacket according to Claim 2 including at least one of an outer layer of insulation operably connected to the outer wall of the body portion, and an inner layer of insulation operably connected to the inner wall of the body portion.

4. An insulation jacket according to Claim 2 or Claim 3 wherein at least one of the layers of insulation is comprised of a closed cell insulating foam.

5. An insulation jacket according to any preceding Claim wherein the body portion has the general shape of a heart, a liver, a lung, a pancreas, or a kidney.

6. An insulation jacket according to any preceding Claim wherein the body portion is constructed from a flexible surgical grade plastic.

7. An insulation jacket according to any preceding Claim wherein the cooling material is a non-toxic sterile gel.

8. An insulation jacket according to any preceding Claim wherein the cooling material is sterile saline.

9. An insulation jacket according to any preceding Claim including a plurality of interconnecting bands positioned between the plurality of pillows, the plurality of interconnecting bands configured to assist in the ability of the insulation jacket to be manipulated about at least a portion of the graft organ.

10. An insulation jacket according to any preceding Claim wherein the body portion is configured to form at least one opening when enclosed about at least a portion of the graft organ, said at least one opening positioned to allow at least one vessel of the graft organ to pass out of the insulation jacket.

11. An insulation jacket according to any preceding Claim wherein the body portion is comprised of a plurality of panels.

12. An insulation jacket according to any preceding Claim wherein the plurality of connectors includes mating strips of hook and loop material.

13. An insulation jacket for thermally affecting a graft organ during harvesting and transplantation operations comprising:
a) a body portion having an inner wall and an outer wall, the body portion generally configured in the shape of the graft organ, the body portion configured to generally conform to the shape of said graft organ when said body portion is secured about at least a portion of said graft organ, the body portion also configured to provide an opening through which at least one vessel of said graft organ may extend away from said body portion when said body portion is enclosed about at least a portion of the graft organ; and
b) a plurality of connectors, the plurality of connectors operably connected to the insulation jacket, the plurality of connectors being configured to secure the insulation jacket about at least a portion of the graft organ.

14. An insulation jacket according to Claim 13 wherein said body portion is comprised of an insulation foam.

15. An insulation jacket according to Claim 14 wherein the insulation is a closed cell foam.

16. An insulation jacket according to any of Claims 13 to 15 including a liner, the liner and body portion being configured to form a plurality of pillows configured to retain a cooling material, wherein the body portion is configured to prevent said cooling material from leaking through said body portion.

17. An insulation jacket according to any of Claims 13 to 16 wherein said body portion is comprised of a thermo mass.

18. An insulation jacket according to any of Claims 13 to 16 wherein the body portion is comprised of a surgical grade plastic.

19. An insulation jacket according to any of Claims 13 to 18 wherein at least one of the inner wall and the outer wall is operably connected to at least one layer of insulation.

20. An insulation jacket according to any of Claims 13 to 19 wherein the inner wall is operably connected to the outer wall to form a plurality of non-communicating pillows, the plurality of non-communicating pillows having an inner section, and being configured to contain a cooling material, which cooling material is non-toxic and sterile, and capable of having a malleable nature when cooled to approximately 4 degrees Celsius.

21. A method for insulating a graft organ during harvesting and transplantation operations comprising:
a) selecting an insulation jacket having the general configuration of the graft organ, the insulation jacket having a body portion, the body portion including a plurality of pillows, the plurality of pillows having an inner section, said inner section having a non-toxic sterile cooling material;
b) chilling the non-toxic sterile cooling material contained within the insulation jacket to between approximately 2° to 4° Celsius, said non-toxic sterile cooling material contained within said inner section maintaining a malleable condition when chilled to approximately 2° to 4° Celsius;
c) placing the insulation jacket inside a patient;
d) securing the insulation jacket about at least a portion of the graft organ, the insulation jacket having at least one opening configured to allow at least one vessel of the graft organ to pass through the insulation jacket and allow the surgeon access to said at least one vessel for detachment from, or reattach ment to, the patient;
e) electing whether to puncture at least one of the plurality of pillows; and
f) removing the insulation jacket from the body of the patient, said removal occurring after the graft organ has been transplanted or harvested.

22. A method of insulating a graft organ in transit from the donor to a recipient, comprising securing an insulation jacket according to any of Claims 1 to 20 about at least a portion of the organ.

23. A method according to Claim 22 including the step of chilling the cooing material in the jacket to a temperature in the range 2° to 4° Celsius.
